# EUROPEAN PATENT APPLICATION

(11) **EP 4 723 035 A1**
(43) Date of publication of application: **08.04.2026**
(21) Application number: 25201847.8
(22) Date of filing: 12.09.2025
(51) Int. Cl.: G06T 7/00, A61B 6/46

(54) **A COMPOSITE RENDERING INCLUDING THE CORONARY ARTERIES, PLAQUE, AND COMPUTED TOMOGRAPHY-FRACTIONAL FLOW RESERVE (CT-FFR) INFORMATION**

(30) Priority: 07.10.2024 US 202418907648
(71) Applicant: GE Precision Healthcare LLC, Waukesha, WI 53188 (US)
(72) Inventor: TOULEMON, Florentin, Waukesha, 53188-1696 (US); AMICE, Romane, Waukesha, 53188-1696 (US); DURANT, Jérôme, Waukesha, 53188-1696 (US); DEUBIG, Amy, Waukesha, 53188-1696 (US); SMITH, Christine, Waukesha, 53188-1696 (US); PACK, Jed, Waukesha, 53188-1696 (US); DAVIS, Cynthia, Waukesha, 53188-1696 (US); FANARIOTIS, Michail, Waukesha, 53188-1696 (US)
(74) Representative: Kilburn & Strode LLP

(57) **Abstract**

A computer-implemented method includes obtaining an image of one or more anatomical vessels of a subject (1202), wherein the image is a segmentation of the one or more anatomical vessels from image data acquired with a three-dimensional medical imaging modality. The computer-implemented method further includes obtaining one or more segmentations of plaque in the one or more anatomical vessels of the subject (1204). The computer-implemented method further includes obtaining Fractional Flow Reserve (FFR) information for the one or more anatomical vessels of the subject (1206). The computer-implemented method further includes generating a composite rendering that includes the image of one or more anatomical vessels, at least one of the one or more segmentations of plaque, and the FFR information (1208). The computer-implemented method further includes displaying the composite rendering in a single viewport.

## Description

### FIELD

The following generally relates to Computed Tomography-Fractional Flow Reserve (CT-FFR), and more particularly to a composite rendering that includes a coronary artery tree, plaque, and CT-FFR information.

### BACKGROUND

A computed tomography (CT) scanner includes a gantry and a rotating frame rotatably supported by a bearing in the gantry. The rotating frame is configured to rotate around an examination region along an axis of rotation about a center of rotation (i.e., an isocenter). The rotating frame carries components such as an X-ray source, an X-ray radiation sensitive detector array, a Data Acquisition System (DAS), etc. For axial and/or helical scanning, the rotating frame rotates around the examination region, the X-ray source emits X-ray radiation that traverses the isocenter (and a subject and/or object in the examination region), and is detected by the X-ray radiation sensitive detector array.

The DAS generates and outputs projection data (line integrals) indicative of the sensed X-ray radiation. A reconstructor reconstructs the projection data and generates three-dimensional (3-D) volumetric image data. Voxels of the reconstructed volumetric image data are displayed as a two-dimensional (2-D) CT image or slice and/or a three-dimensional (3-D) rendering, both at least using gray scale values corresponding to a relative radiodensity. The gray scale values reflect the attenuation characteristics of the scanned subject and/or object and generally show structure such as anatomical structures within the scanned subject and/or physical structure within the scanned object.

Fractional flow reserve (FFR) is a technique utilized to measure pressure differences across a coronary artery stenosis. The literature defines FFR as the pressure after a stenosis relative to the pressure before the stenosis and indicates an FFR expresses a maximal flow down a vessel in a presence of a stenosis compared to a maximal flow in a hypothetical absence of the stenosis. An FFR value is a number from 0.0 to 1.0 representing a percentage drop in blood pressure due to the stenosis (e.g., a value of 0.60 indicates that a given stenosis causes a 30% drop in blood pressure, etc.). The literature indicates that an FFR of 0.80 represents functional significance, and an FFR of 0.70 represents severe pathological blood pressure.

FFR has been determined invasively during a coronary catheterization procedure. For this, a catheter is inserted into the femoral or radial arteries and advanced to the stenosis, where a sensor at the tip of the catheter senses pressure, temperature, and flow across the stenosis, during conditions promoted by various agents that affect vessel geometry, compliance and resistance, and/or other characteristics. A non-invasive approach includes Computed Tomography-Fractional Flow Reserve (CT-FFR), which estimates an FFR from CT image data of the heart that was acquired during a contrast enhanced coronary computed tomography angiography (CCTA) procedure using computational fluid dynamic (CFD) simulations in which blood flow and pressure through the coronary arteries are simulated.

CT-FFR results have been superimposed over an anatomical rendering of a 3-D segmentation of the coronary artery tree using a color gradient. The literature indicates that the color blue represent "normal" FFR values (or values greater than 0.80), the colors orange to yellow represent "borderline" FFR values (or values of 0.76 to 0.80), and the color red represents "abnormal" FFR values (or value less than 0.76), again, where a value of 0.70 indicates severe pathological blood pressure. This rendering is displayed in a viewport with other viewports displaying other information such as a 2-D image, a curved Multiplanar Reconstruction (MPR), a 3-D Maximum Intensity Projection (MIP), etc. that show stenoses and plaque composition (i.e., soft / non-calcified plaque, hard / calcified plaque, and/or mixed plaque, which includes both soft and hard plaque).

Such a display separates some anatomical and functional information across different viewports. As a consequence, the clinician evaluating a cardiac heath state of the patient has to read different renderings displayed in different viewports and mentally correlate the information across the viewports in an attempt to piece together the different information to gain an understanding of the cardiac health state of the patient. Unfortunately, the process of mentally correlating such information across different viewports is dependent on the clinician reviewing the information (e.g., based on their experience, field of expertise, etc.) and is subject to human error which may lead to inaccuracies to the detriment of the patient, consumes the clinician's time which could be otherwise utilized with the patient and/or another patient, is inefficient, etc.

In view of at least the foregoing, there is an unresolved need for an improved approach for presenting CT-FFR results with other renderings.

### SUMMARY

Aspects described herein address the above-referenced problems and others. This summary introduces concepts that are described in more detail in the detailed description. It should not be used to identify essential features of the claimed subject matter, nor to limit the scope of the claimed subject matter.

In one aspect, a computer-implemented method includes obtaining an image of one or more anatomical vessels of a subject, wherein the image is a segmentation of the one or more anatomical vessels from image data acquired with a three-dimensional medical imaging modality. The computer-implemented method further includes obtaining one or more segmentations of plaque in the one or more anatomical vessels of the subject. The computer-implemented method further includes obtaining Fractional Flow Reserve (FFR) information for the one or more anatomical vessels of the subject. The computer-implemented method further includes generating a composite rendering that includes the image of one or more anatomical vessels, at least one of the one or more segmentations of plaque, and the FFR information. The computer-implemented method further includes displaying the composite rendering in a single viewport.

In another aspect, a system includes a medical imaging modality configured to acquire projection data and generate three-dimensional image data of an interior region of a subject, a display device and an operator console with processor. The processor is configured to segment the three-dimensional image data to generate an image of one or more vessels of a subject, segment the three-dimensional image data or the image to generate one or more segmentations of plaque in the one or more anatomical vessels, determine FFR information for the one or more anatomical vessels from the three-dimensional image data, generate a composite rendering that includes the image of one or more anatomical vessels, the one or more segmentations of plaque, and the FFR information, and display the composite rendering in a single viewport visually presented on the display device.

In another aspect, a computer readable medium is encoded with computer executable instructions. The computer executable instructions, when executed by a processor, cause the processor to acquire projection data and generate three-dimensional image data of an interior region of a subject with a medical imaging modality, segment an image of one or more vessels of a subject from the three-dimensional image data, segment one or more segmentations of plaque in the one or more anatomical vessels from the three-dimensional image data or the image, determine FFR information for the one or more anatomical vessels with the three-dimensional image data, generate a composite rendering that includes the image of one or more anatomical vessels, the one or more segmentations of plaque, and the FFR information, and display the composite rendering in a single viewport visually presented on a display device.

Those skilled in the art will recognize still other aspects of the present application upon reading and understanding the attached description.

### BRIEF DESCRIPTION OF THE DRAWINGS

The application is illustrated by way of example and not limited by the figures of the accompanying drawings in which like references indicate similar elements.

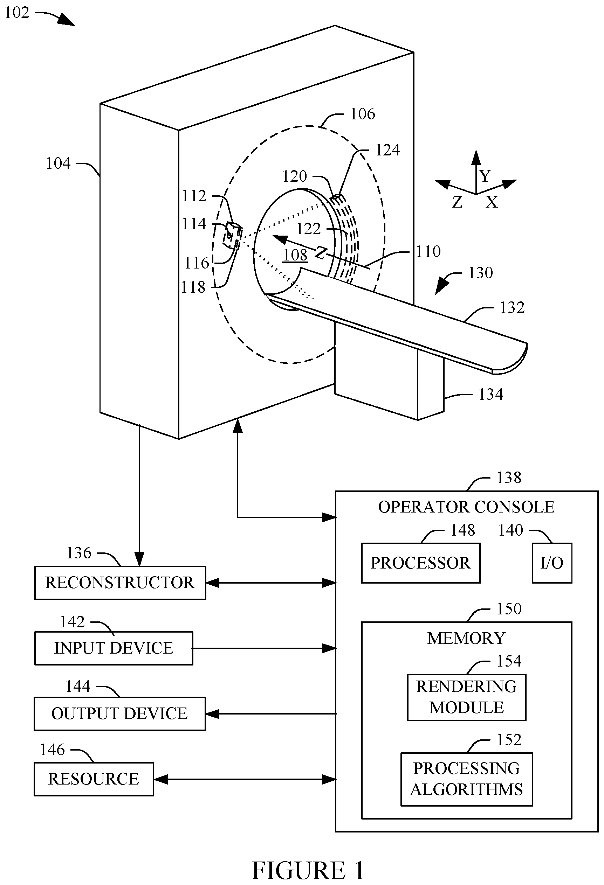

FIGURE 1 schematically illustrates a non-limiting example of an imaging system configured for computed tomography imaging and including processing algorithms and a rendering module configured to generate a single composite rendering that includes a coronary artery tree segmentation, a plaque segmentation(s), and CT-FFR information, in accordance with an embodiment(s) herein.

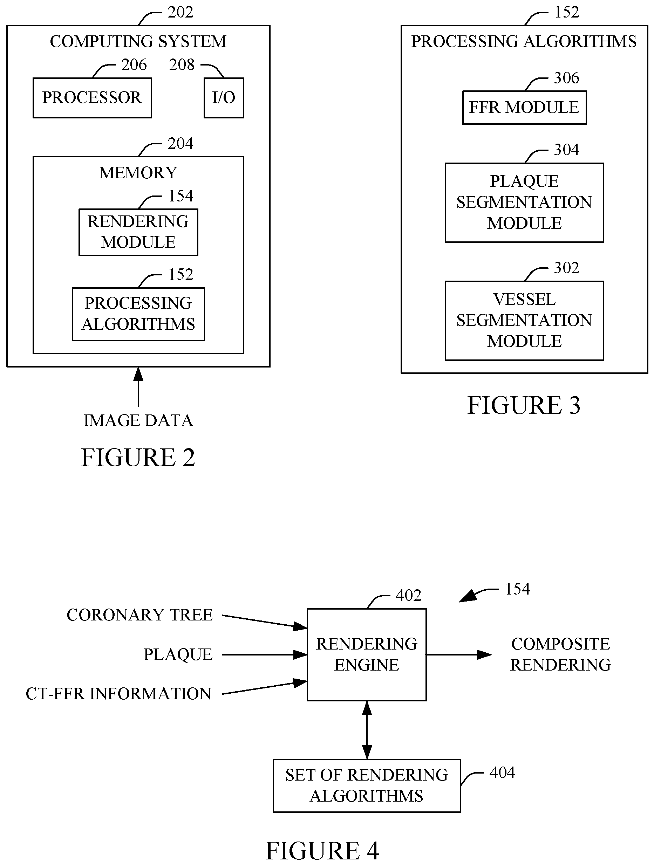

FIGURE 2 schematically illustrates a non-limiting example of a computing system including the processing algorithms and/or the rendering module configured to generate the single composite rendering that includes the coronary artery tree segmentation, the plaque segmentation(s), and the CT-FFR information, in accordance with an embodiment(s) herein.
FIGURE 3 schematically illustrates a non-limiting example of the processing algorithms of FIGURES 1 and 2, in accordance with an embodiment(s) herein.
FIGURE 4 schematically illustrates a non-limiting example of the rendering module of FIGURES 1 and 2, in accordance with an embodiment(s) herein.

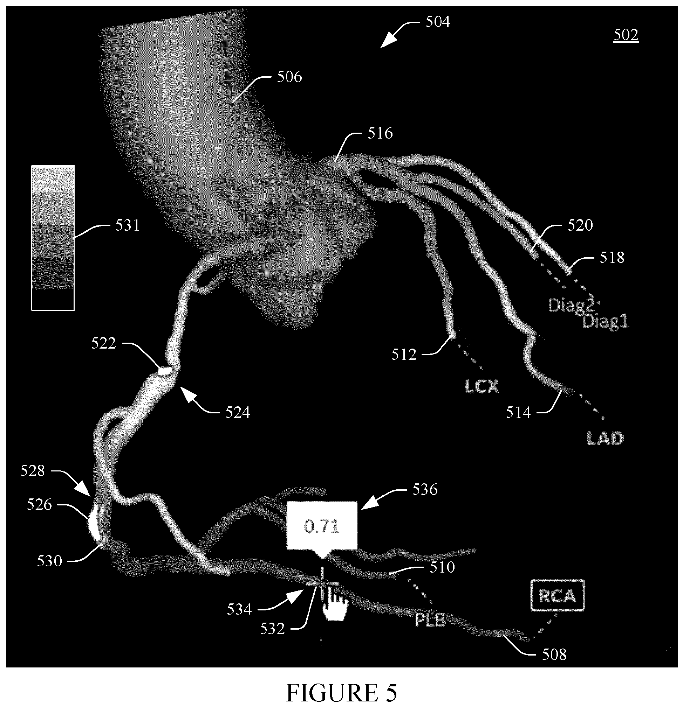

FIGURE 5 graphically depicts an example single composite rendering that includes a coronary artery segmentation, multiple plaque segmentations, and CT-FFR information where the multiple plaque segmentations are overlaid over regions in a rendering of an interior of vessels of the coronary artery segmentation that is mapped to the CT-FFR information via a gray scale mapping, in accordance with an embodiment(s) herein.

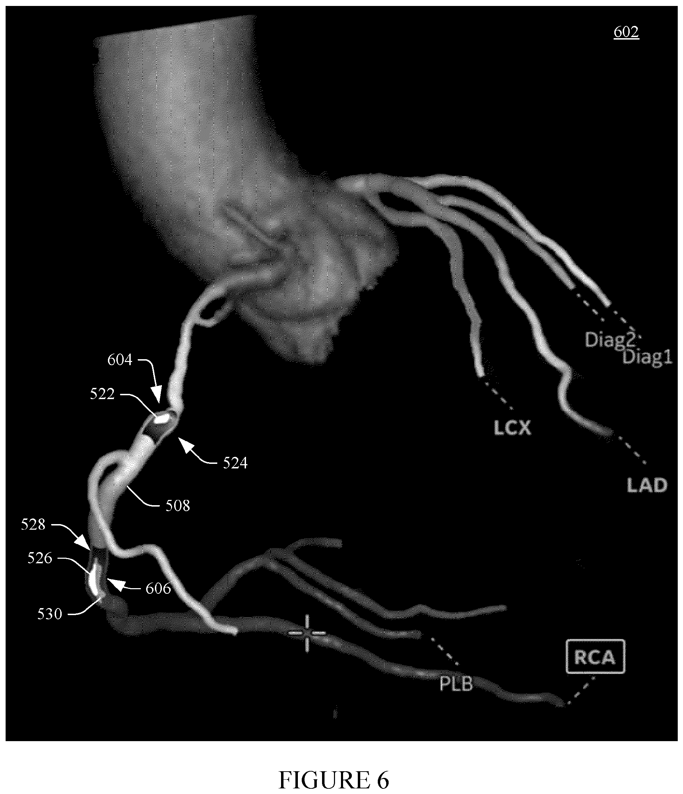

FIGURE 6 graphically depicts an example single composite rendering that includes the coronary artery tree segmentation, the multiple plaque segmentations, and the CT-FFR information where the multiple plaque segmentations are overlaid over cut outs in vessels of the coronary artery tree segmentation inside of the vessels and the coronary artery tree segmentation is mapped to the CT-FFR information via a gray scale mapping, in accordance with an embodiment(s) herein.

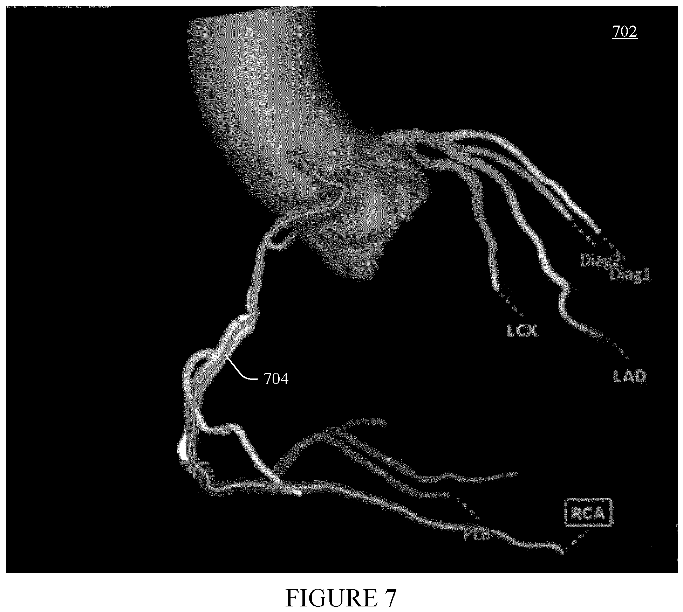

FIGURE 7 graphically depicts an example single composite rendering that includes the coronary artery tree segmentation, the multiple plaque segmentations, and the CT-FFR information where the multiple plaque segmentations where the multiple plaque segmentations are overlaid over regions in a rendering of an interior of vessels of the coronary artery segmentation that is mapped to the CT-FFR information via a gray scale mapping, additionally with a centerline of the right coronary artery of the coronary artery tree segmentation superimposed over the right coronary artery, in accordance with an embodiment(s) herein.

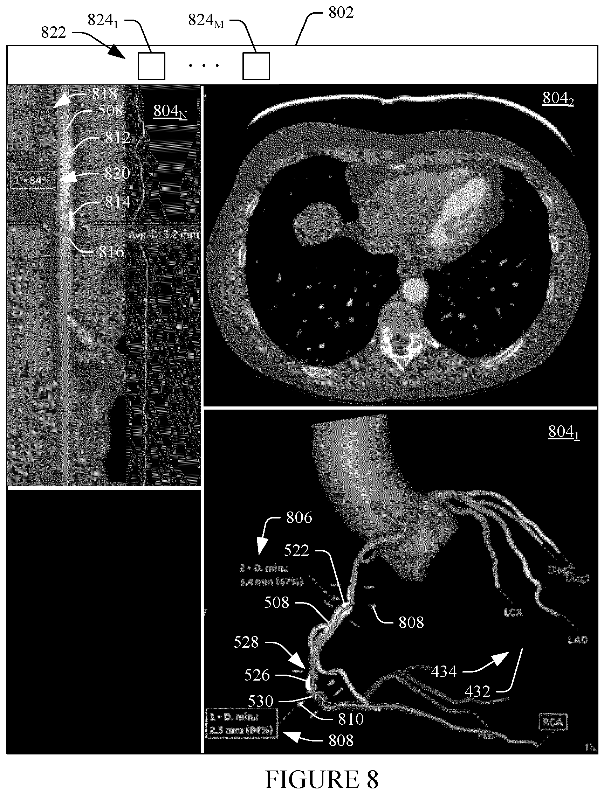

FIGURE 8 graphically illustrates an example graphical user interface displaying the composite rendering of FIGURE 7 along with annotations in a dedicated viewport and other viewports displaying other cardiac image data, in accordance with an embodiment(s) herein.

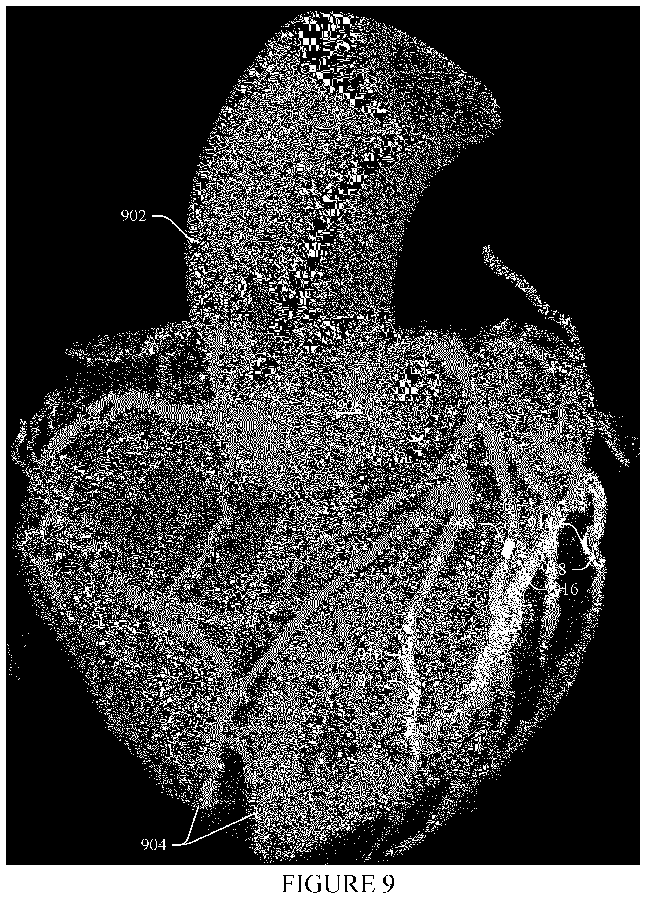

FIGURE 9 graphically depicts an example combined rendering that includes a composite rendering, which includes a coronary artery tree segmentation, a plaque segmentation(s), and CT-FFR information, with another anatomical segmentation, in accordance with an embodiment(s) herein.

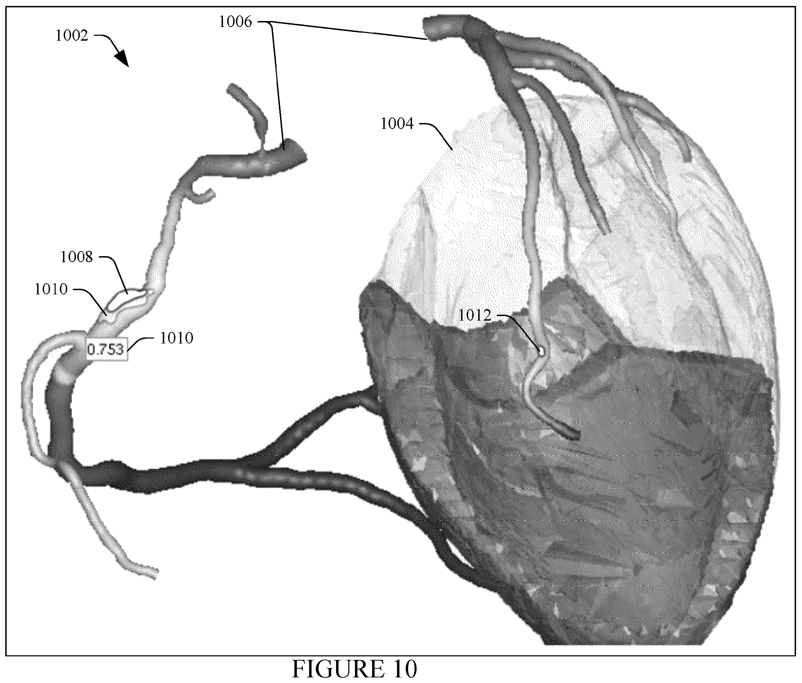

FIGURE 10 graphically depicts an example combined rendering that includes a composite rendering, which includes a coronary artery tree segmentation, plaque segmentation(s), and CT-FFR information, with another anatomical segmentation, in accordance with an embodiment(s) herein.

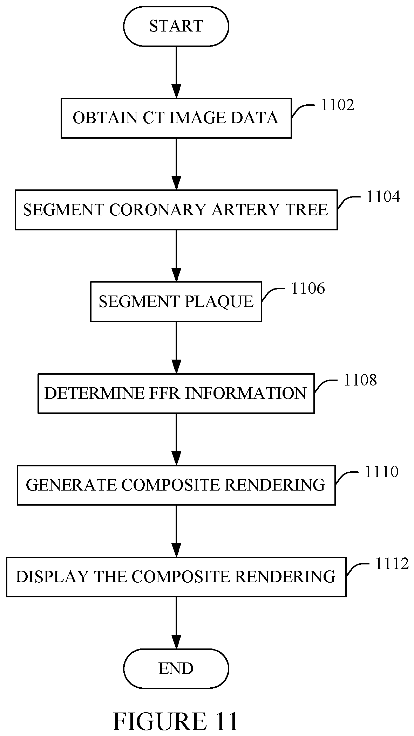

FIGURE 11 illustrates a non-limiting example of a flow chart for generating a composite rendering that includes a coronary artery tree segmentation, plaque segmentations, and CT-FFR information, where the plaque segmentations are overlaid over regions in a rendering of an interior of vessels of the coronary artery segmentation or inside of cut outs of the vessels, and the CT-FFR information is mapped to the vessels via a gray scale mapping, in accordance with an embodiment(s) herein.

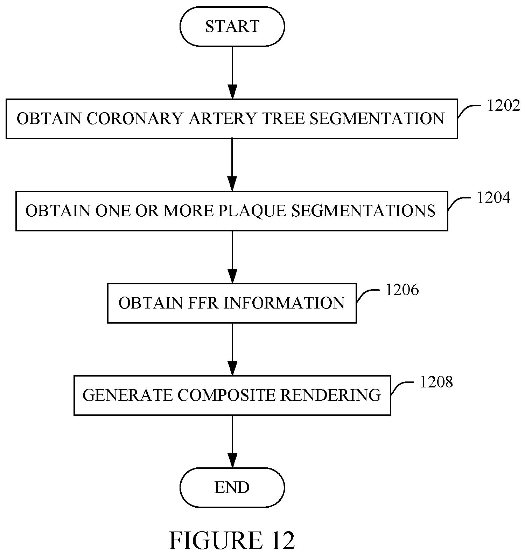

FIGURE 12 illustrates a non-limiting example of a flow chart for generating a composite rendering that includes a coronary artery tree segmentation, plaque segmentations, and CT-FFR information, where the plaque segmentations are overlaid over regions in a rendering of an interior of vessels of the coronary artery segmentation or inside of cut outs of the vessels, and the CT-FFR information is mapped to the vessels via a gray scale mapping, in accordance with an embodiment(s) herein.

### DETAILED DESCRIPTION

Embodiments of the present disclosure will now be described, by way of example, with reference to the figures, in which a system, a method and/or instructions of a computer readable medium visually presents certain anatomical and functional information in a single view, including, in one instance, a single composite rendering that includes a coronary artery tree segmentation, a plaque segmentation(s), and CT-FFR information. Such a rendering, which merges anatomical and physiological information, provides an efficient and comprehensive overview that can be used to analyze each patient condition to diagnose, to guide treatment strategy, to plan and guide cardiac procedures, etc., where the composite rendering provides the anatomical structures of interest (e.g., the coronary artery tree and plaque (hard, soft and/or mixed) and blood pressure information in a single view).

As discussed above, CT-FFR results have been displayed as a rendering that includes a 3-D segmentation of the coronary tree with an FFR color gradient superimposed thereover, where colors of the color gradient correspond to a health state from "normal" to "abnormal," including functionally significant to severe pathological blood pressure. Along with this display, other information, such as an axial image, a curved MPR, a MIP, etc. that shows stenosis and plaque composition (e.g., soft, hard, and/or mixed), are displayed in other viewports. The clinician evaluating a cardiac health state of the patient then has to read different renderings displayed in different viewports and mentally correlate the information in an attempt to piece together the information to gain an understanding of the cardiac health state of the patient. As discussed herein, the process of mentally correlating such information is different viewports is dependent on the clinician reviewing the information and is subject to human error which may lead to inaccuracies to the detriment of the patient, consumes the clinician's time which could be otherwise utilized with the patient or another patient, is inefficient, etc.

The single composite rendering described herein mitigates having to have the clinician read different renderings and mentally correlate the different anatomical and functional renderings to piece together the health state of the patient, and, thus, mitigates the inaccuracies, time consumption, inefficiencies, etc. associated therewith, providing an improvement to the technology. In one instance, the composite rendering integrates all the clinical information required in a single view, including a blood pressure overview vis FFR information, calcified plaques and/or soft plaque size and distribution, in a fully integrated and seamless workflow, which can be utilized to diagnose a cardiac state of a patient, guide a treatment strategy, plan and guide a cardiac procedure such as Percutaneous Coronary Intervention (PCI) and/or a surgical revascularization (Coronary Artery Bypass Graft). The plaque segmentation can be variously displayed, e.g., merged with a rendering of an interior of the coronary tree and the CT-FFR gradient, within the coronary tree using a sectional view of a vessel at a location of plaque in the vessel, etc., and the composite rendering can be combined with another anatomical segmentation, providing a multi-object rendering of anatomical and functional information.

Initially referring to FIGURE 1, a non-limiting example of an imaging system 102 such as a computed tomography (CT) imaging system is schematically illustrated. The imaging system 102 includes a gantry 104. In some instances, the gantry 104 is configured to tilt. The imaging system 102 further includes a rotating frame 106. The rotating frame 106 is rotatably supported in the gantry 104, e.g., via a bearing (e.g., a slip ring) or the like, and is configured to rotate around an examination region 108 about a rotational or z-axis 110, which extends through a center of rotation / a center of the examination region 108 (i.e., an isocenter). A gantry controller (not visible) is configured to control rotation of the rotating frame 106 and, if configured to tilt, tilting of the gantry 104.

An X-ray source assembly 112 is supported by the rotating frame 106 and rotates in coordination with the rotating frame 106. The X-ray source assembly 112 includes an X-ray source 114 such as an X-ray tube. The X-ray source 114 is configured to emit X-ray radiation having an energy in the X-ray diagnostic range (e.g., 20 keV to 150 keV). The X-ray source assembly 112 may further include or is coupled to a filter 116 that characterizes an X-ray radiation dose profile and/or a collimator 118 that shapes the X-ray radiation to form a generally fan, wedge, cone, etc. shaped beam that traverses the examination region 108. An X-ray controller (not visible) is configured to control components of the X-ray assembly 112 such as X-ray radiation emission of the X-ray source 114, the collimator 118, etc.

An X-ray radiation sensitive detector array 120 includes a one-dimensional (1-D) or two-dimensional (2-D) array of rows of X-ray radiation sensitive detector elements 122 and is supported by the rotating frame 106 along an arc opposite the X-ray source 114, across the examination region 108. Each of the X-ray radiation sensitive detector elements 122 is in electrical communication with a data acquisition system (DAS) 124. The X-ray radiation sensitive detector elements 122 include an indirect conversion detector such as a scintillator / photodiode detector and/or a direct conversion detector such as a Cadmium Telluride (CdTe), a Cadmium Zinc Telluride (CZT), etc. detector. A DAS controller (not visible) controls the X-ray radiation sensitive detector array 120.

A subject/object support 130 includes a tabletop 132 moveably coupled to a frame/base 134. In one instance, the tabletop 132 is slidably coupled to the frame/base 134 via a bearing or the like, and a drive system (not visible) including a controller, a motor, a lead screw, and a nut (or other drive system) translates the tabletop 132 along the frame/base 134 into and out of the examination region 108. The tabletop 132 is configured to support an object or subject in the examination region 108 for loading, scanning, and/or unloading the subject or object. A table controller (not visible) controls the drive system.

For an axial scan, the tabletop 132 is positioned at a static position for each integration period and moves between integration periods. For a helical scan, the rotating frame 106 rotates in coordination with the tabletop 132 moving along the Z-axis 110, and active X-ray detector elements 122 of the X-ray radiation sensitive detector array 120 detect X-ray radiation over consecutive arc segments (integration periods) each revolution and generate respective signals. For each arc segment, the data acquisition electronics (DAS) 124 processes each signal and generates projection data. For an image acquisition such as a Coronary CT angiography (CCTA), which is used to assess the coronary arteries of the heart, a radio opaque contrast agent is first administered to the patient and then the heart of the patient is scanned.

A reconstructor 136 reconstructs the projection data and generates volumetric three-dimensional (3-D) image data for a helical scan and/or individual axial two-dimensional (2-D) images for an axial step and shoot scan (which can be used in combination to generate volumetric image data). The volumetric image data and/or 2-D slices thereof, and/or the individual axial images can be visually presented, filmed, etc. Examples of suitable reconstruction algorithms include filtered back projection (FBP), advanced statistical iterative reconstruction (ASIR), conjugate gradient (CG), maximum likelihood expectation maximization (MLEM), model-based iterative reconstruction (MBIR), and/or other reconstruction algorithm.

With a non-spectral configuration, the X-ray source 114 includes a single broadband X-ray tube that emits polychromatic radiation, e.g., 40 keV to 120 keV at 120 kVp. In another example, the imaging system 102 is configured for spectral imaging. With such a configuration, the X-ray source 114 may be configured to switch between at least two different kVp values, include multiple X-ray tubes, etc., and/or the detector array 120 may include multiple layers, each configured to detect a different energy, etc., where the projection data can be decomposed into photoelectric effect and Compton scattering components, and the reconstructor 136 can reconstruct projection data for different energy bands.

A computing system 138 serves as an operator console of the system 102. The computing system 138 may include a computer, a workstation, etc. The computing system 138 includes input/output (I/O) 140. An input device 142 includes a keyboard, mouse, touchscreen, microphone, etc. The input device 142 is in electrical communication with the computing system 138 through the I/O 140 and/or otherwise. An output device 144 includes a human readable device such as a display monitor or the like. The output device 144 is in electrical communication with the computing system 138 through the I/O 140 and/or otherwise.

A remote resource 146 includes one or more of a server, a workstation, a Radiology Information System (RIS), a Hospital Information System (HIS), an electronic medical record (EMR), a Picture Archiving and Communications System (PACS), one or more other CT scanners, cloud processing resources (which includes shared remote data storage and/or computing power, including processing resources distributed over multiple locations / data centers), etc. The remote resource 146 is in electrical communication with the computing system 138 through the I/O 140 and/or otherwise. In general, the remote resource 146 includes a device that is not part of the imaging system 102, but is configured to communicate with the imaging system 102.

The computing system 138 further includes at least one processor 148 such as a microprocessor (µP), a central processing unit (CPU), graphics processing unit (GPU), etc., and a computer readable medium 150 ("MEMORY"), which includes non-transitory medium and excludes transitory medium (signals, carrier waves, and the like). The at least one processor 148 is configured to execute application software that allows a user to set up and start a scan (e.g., select an image protocol, enter a scan parameter, initiate scanning, etc.) and view image data. For CT-FFR, the selected protocol may include a CCTA protocol configured to acquire data for assessing the arteries of the heart, such as an extent of occlusion in the coronary arteries, etc.

The computer readable medium 150 at least includes processing algorithms 152 and a rendering module 154. As described in greater detail below, in one instance the processing algorithms 152 are configured at least to segment a coronary artery tree from image data, segment plaque from the coronary tree and/or the image data, and determine FFR information for the vessels of the coronary artery tree, and the rendering module 154 is configured at least to generate a single composite rendering that includes the coronary artery tree segmentation, one or more plaque segmentations, and the FFR information, all in a single view. As discussed herein, the single composite rendering mitigates having to have the clinician read different renderings and mentally correlate the different anatomical and functional renderings to piece together the health state of the patient, and, thus, mitigates the inaccuracies, time consumption, inefficiencies, etc. associated therewith, for a configuration that does not include the rendering module 154.

In FIGURE 1, the processing algorithms 152 and the rendering module 154 are included in the memory 150 of the operator console 138 of the imaging system 102. Additionally, or alternatively, the processing algorithms 152 and/or the rendering module 154 are included in a memory of one or more other computing systems. FIGURE 2 schematically illustrates an example in which the processing algorithms 152 and the rendering module 154 are included in a computing system 202, e.g., in a memory 204 thereof, that also includes a processor 206 and I/O 208, which are similar to the processor 148 and I/O 140 described in connection with FIGURE 1 and thus will not be described in detail here.

By further way of non-limiting example, in one instance the remote resource 146 includes a PACS (e.g., where the computing system 202 is a PACS), and the PACS includes and is configured to execute the processing algorithms 152 and/or the rendering module 154. In another instance, the processing algorithms 152 and the rendering module 154 are distributed across and executed by different systems, including instances in which different modules (described below) of the processing algorithms 152 are executed on a same system or at least two different systems. Additionally, or alternatively, the processing algorithms 152 and/or the rendering module 154 is part of a cloud processing resource.

Turning now to FIGURE 3, an example of the processing algorithms 152 (FIGURE 1) is schematically illustrated. The example processing algorithms 152 includes at least a vessel segmentation module 302, a plaque segmentation module 304, and an FFR module 306.

The vessel segmentation module 302 includes at least a segmentation algorithm configured to segment a coronary artery tree from image data. In one instance, the image data is generated by the imaging system 102. In this instance, the vessel segmentation module 302 obtains the image data from the reconstructor 136, the memory 150, and/or a resource of the resource 146. In another instance, the vessel segmentation module 302 obtains image data generated by another imaging system, e.g., via a resource of the resource 146, from the other imaging system, etc.

The vessel segmentation module 302 receives, as input, image data and segments the coronary artery tree from the image data via the at least one vessel segmentation algorithm. In one instance, the segmentation is performed automatically, e.g., machine learning, etc. In another instance, the segmentation is performed semi-automatically, e.g., with user assistance. In one instance, the segmentation includes identifying and/or extracting coronary artery centerlines and/or lumen geometry (e.g., diameter, perimeter, cross-sectional area, etc.) therefrom. The segmentation can be based on voxel intensity, object shape, and/or other characteristics.

Examples of suitable approaches for segmenting the coronary tree are described in patent EP3809376A2 to Knoplioch et al., entitled "Systems and Methods for Visualizing Anatomical Structures," and filed September 29, 2020, US10186056B1 to Senzig et al., entitled "System and Method For Estimating Vascular Flow Using CT imaging," and filed March 1, 2012, and US10964017B1 to Pack et al., entitled "Deep Learning For Arterial Analysis and Assessment," and filed November 5, 2018. EP3809376A2 describes an approach that processes the image data into segments of a vessel, including one or more segments corresponding to a vessel wall, one or more segments corresponding to tissue, one or more segments corresponding to plaque, etc. The approach also calculates or measures a position of a center line of the vessel. In one instance, the center line is determined by measuring a radial distance from the walls of the vessel, and, in some examples, and determining a set of points located in the center of the vessel. The approach also is configured to generate a cut mask based on one or more cut angles and the center line to provide a view showing an inside of a portion of the vessel by applying the cut mask, where voxels therein are rendered transparent or partially transparent.

The plaque segmentation module 304 receives, as input, the segmented coronary artery tree and/or the image data, and segments plaque identified therein via a plaque segmentation algorithm. Examples of suitable approaches for segmenting plaque are described in patent US8077939B2 to Nezet et al., entitled "Methods and systems for enhanced plaque visualization," and filed November 22, 2006, and US10186056B. US8077939B2 describes an embodiment that classifies at least one region in a visual representation of the vessel as a plaque region. The vessel is further segmented into one or more vessel regions based on the classification, e.g., the vessel can be segmented into lumen, soft plaque, calcified plaque, and/or background. US10186056B describes an embodiment that performs plaque segmentation around a contrast-enhanced lumen of the coronary arteries.

The FFR module 306 receives, as input, the segmented coronary tree and determines FFR information such as FFR values for the segmented coronary tree. An example of a suitable approach for determining FFR values is described in patent US9949650B1to Edic et al., entitled "Fractional Flow Reserve Estimation," and US10964017B1. US9949650B1 describes an approach that employs a CFD module to compute a transstenotic pressure difference: ΔP = Pₐₒᵣₜₐ -P_{distal}, or a ratio of the two pressures, where if a pressure in the aorta is non-invasively estimated, the transstenotic pressure difference can be subtracted from the aortic pressure to estimate the distal coronary pressure, and, with these two quantities, an FFR can then be computed, where, in one implementation, the mean arterial blood pressure in the brachial artery, estimated with a standard blood pressure cuff, is used to approximate the mean aortic pressure, either as is or modified with a population-relevant, demographic-relevant scaling factor. US10964017B1 describes a deep learning approach.

Moving to FIGURE 4, an example of the rendering module 154 (FIGURE 1) is schematically illustrated. The example of the rendering module 154 includes a rendering engine 402 and a set of rendering algorithms 404. The rendering engine 402 receives, as input, at least a segmented coronary artery tree, one or more plaque segmentations, and CT-FFR information. In instances in which the composite rendering is combined with other anatomical segmentations, the rendering engine 402 further receives, as input, image data and generates the other anatomical segmentations and/or the other segmentations.

In one instance, the rendering engine 402 receives the image data generated by the reconstructor 136, the coronary artery tree segmented by the vessel segmentation module 302, the one or more plaque segmentations segmented by the plaque segmentation module 304, CT-FFR information generated by the FFR module 306. In another instance, at least one of the image data, the segmented coronary artery tree, the one or more plaque segmentations, and the CT-FFR information is otherwise generated and/or obtained.

The set of rendering algorithms 404 includes 2-D and/or 3-D rendering algorithms, including a composite rendering algorithm, which at least generates a composite rendering that at least includes the coronary artery tree segmentation, at least one plaque segmentation, and CT-FFR information mapped to the coronary artery tree segmentation, all in a single view. Again, the single composite rendering mitigates having to have the clinician read different renderings and mentally correlate the different anatomical and functional renderings to piece together the health state of the patient, e.g., in configurations that do not include the rendering module 154, and, thus, mitigates the inaccuracies, time consumption, inefficiencies, etc. associated therewith, providing a technological improvement to existing CT-FFR technology.

FIGURES 5, 6 and 7 graphically depict examples of composite renderings. In FIGURE 5, an interior of the coronary artery tree is rendered with indicia indicating CT-FFR values along the vessels and plaque segmentations superimposed over regions of the interior of the vessels that include plaque. In FIGURE 6, the coronary artery tree is rendered with indicia indicating CT-FFR values and the vessels include cut outs at the regions that include plaque, and the plaque segmentations are disposed inside of the vessels. In FIGURE 7, the coronary artery tree further includes a centerline overlaid over the right coronary artery. The composite renderings of FIGURES 5, 6 and 7 are described in greater detail next.

Initially referring to FIGURE 5, an example composite rendering 502 that graphically combines a coronary artery tree, plaque segmentations, and CT-FFR information is graphically depicted. The composite rendering 502 includes a coronary artery tree segmentation 504. The illustrated coronary artery tree segmentation 504 is a 3-D volume rendering that shows an interior of the vessels. In another instance, the coronary artery tree segmentation 504 is a 2-D rendering. The illustrated segmentation includes at least an aorta 506, a right coronary artery (RCA) 508, a posterolateral branch (PLB) 510, a left circumflex artery (LCX) 512, a left anterior descending (LAD) 514, a left main coronary artery (LMCA) 516, a diagonal branch D1 518, and a diagonal branch D2 520.

The composite rendering 502 further includes a first plaque segmentation 522 superimposed or overlaid at a region 524 in the interior of the RCA 508 where plaque was identified. The composite rendering 502 further includes a second plaque segmentation 526 superimposed or overlaid at a different region 528 in the RCA 508 where plaque was identified. The composite rendering 502 further includes a third plaque segmentation 530 superimposed or overlaid at the different region 528 in the RCA 508. The first plaque segmentation 522 corresponds to hard plaque, the second plaque segmentation 526 also corresponds to hard plaque, and the third plaque segmentation 530 corresponds to soft plaque.

The first plaque segmentation 522 and the second plaque segmentation 526 are rendered using Maximum Intensity Projection (MIP), Scalable Vector Graphics (SVG) rendering, and/or other approach that visibly distinguishes the first and second plaque segmentations 522 and 526 from the RCA 508. The first and second plaque segmentations 522 and 526 are rendered white or near white, however other shades of gray are contemplated herein. The third plaque segmentation 530 is also rendered using MIP, SVG, and/or other approach that visibly distinguishes the third plaque segmentation 530 from the RCA 508 and the first and second plaque segmentations 522 and 526. In the illustrated example, the third plaque segmentation 530 is rendered gray.

The composite rendering 502 further includes a gray scale-to-CT-FFR value mapping superimposed or overlaid over the coronary tree segmentation 504. A gray scale legend 531 indicates lighter shades of gray correspond to healthier tissue and darker shades of gray correspond to less healthy tissue. In one instance, the lightest shade of gray corresponds to an CT-FFR value of 1.0, the darkest shade of gray corresponds to a CT-FFR value of 0.70 or less, and shades of gray therebetween correspond to value between 1.0 and 0.70. In the example, the gray scale shade darkens between the first and second plaque segmentations 522 and 526, and further darkens after the second and third plaque segmentations 526 and 530, indicating blood pressures changes after the plaques 522, 526 and 530.

Other visibly distinguishable indicia is also contemplated herein. By way of non-limiting example, color, patterns, and/or other graphical indicia can be alternatively, or additionally, utilized to visually distinguish CT-FFR values along one or more vessels of the coronary artery tree segmentation 504. With one color scheme, the color blue corresponds to a CT-FFR value of 1, which indicates of a "normal" blood pressure, and the color red corresponds to a CT-FFR value of 0.70, which indicates severe pathological blood pressure. This color code is known in the art where blue generally refers to "good" results and red generally refers to "bad" results. However, different colors and/or gradient are contemplated herein.

As described in greater below, the composite rendering 502, in one instance, is displayed in a single dedicated viewport of a graphical user interface (GUI) of an output device such as a display monitor of the output device 144 (FIGURE 1) and/or the resource 146 (FIGURE 1). In one instance, the viewport is part of an executing image processing application software of the operator console 138 (FIGURE 1) and/or the resource 146 (FIGURE 1). Where the viewport is part of an executing image processing application software of the operator console 138, a user input device such as a mouse, touch screen, or the like of the input device 142 (FIGURE 1) can be configured to receive a user input requesting display of a numerical CT-FFR value at a location of interest in the coronary tree segmentation 506.

By way of non-limiting example, in the illustrated embodiment, a visible and movable pointer 532, such as a graphical cursor for an input device such as mouse, touchpad, trackball, etc., is positioned over a region 534 of the RCA 508 and a numerical CT-FFR value 536 is displayed for the region 534. In one instance, the user merely hovers the pointer 532 over the region 534 and the numerical CT-FFR value 536 is displayed. In another instance, the user hovers the pointer 532 over the region 534 and performs another act such as depresses a button of the input device to display the numerical CT-FFR value 536. In one instance, the numerical CT-FFR value 536 is visibly removed once the pointer 532 is no longer over the region 534. In another instance, the numerical CT-FFR value 536 remains visible until the user issues a command to remove the pointer 532.

Turning to FIGURE 6, a composite rendering 602 is substantially similar to the composite rendering 502 of FIGURE 5 except that the RCA 508 is rendered with cut outs 604 and 606 respectively at the regions 524 and 530, with the first plaque segmentation 522 superimposed over the cut out 604 and inside of the region 524 of the RCA 508, and the second and third plaque segmentations 526 and 530 superimposed over the cut out 606 and inside of the region 528 of the RCA 508. In one instance, the user can toggle between the composite rendering 502 and the composite rendering 602. In another instance, the user can concurrently display both the composite rendering 502 and the composite rendering 602. The cut outs 604 and 606 can be determined as described herein and/or otherwise.

Turning to FIGURE 7, a composite rendering 702 is substantially similar to the composite rendering 502 of FIGURE 5 except that the composite rendering 702 further includes a vessel centerline 704 superimposed over the RCA 508. In other instance, a vessel centerline is superimposed over a different branch of the coronary artery tree segmentation. In other instance, multiple vessel centerlines are concurrently superimposed over different branches of the coronary artery tree segmentation. The vessel centerline 704 and/or other vessel centerline can be determined as described herein and/or otherwise.

Moving now to FIGURE 8, an example graphical user interface (GUI) 802 with an example display configuration is graphically depicted. The GUI 802 includes multiple viewports, including a first viewport 804₁, 804₂, ..., and an Nth tool 804_{N}, where N is a positive integer equal to or greater than one. A user can close one of more of the viewports 804₁, 804₂, ..., and an Nth tool 804_{N}. Additionally, or alternatively, the user can open one or more other viewports of the N viewports. Additionally, or alternatively, the user can change the information displayed in one or more other viewports of the N viewports.

In this example, the first viewport 804₁ includes the composite rendering 702, additionally with a first annotation 806 and a second annotation 808. The first annotation 806 includes vessel diameter information about a cross section between a marker pair 810, which is at the first plaque segmentation 522. The second annotation 808 includes vessel diameter information about a cross section between a marker pair 810, which is at the second and third plaque segmentations 526 and 530. In other examples, more, less (including no), and/or different annotations are superimposed over the rendering in the first viewport 804₁. The annotations 806 and 808 further indicate the percent blockage of the vessels at the marker pairs.

The second viewport 804₂ includes an axial slice of the image data. The user, via an input device of the input device 142 (FIGURE 1) can select an image slice to display in the second viewport 804₂, change the image slice displayed in the second viewport 804₂, e.g., via a scroll bar and/or otherwise, change the image orientation from axial to sagittal, coronal, oblique, etc., change the rendering to an MIP, an MPR, a VR, etc. The user can also close the second viewport 804₂. In one instance, the image slice displayed in the second viewport 804₂ is automatically selected, e.g., a first slice of the image data that includes the heart, a last slice of the image data that includes the heart, a middle slice of the slices that include the heart, etc.

The Nth viewport 804_{N} includes a curved MPR of a sub-portion of the RCA 508 that includes a first plaque 812, a second plaque 814 and a third plaque 816, respectively corresponding to the first plaque segmentation 522, the second segmentation 526 and the third plaque segmentation 530. The Nth viewport 804_{N} includes a first annotation 818 corresponding to the first annotation 806 in the first viewport 804₁ and a second annotation 820 corresponding to the second annotation 808 in the first viewport 804₁. In one instance, the system is configured so that a viewport like the viewport 804_{N} is automatically opened by the application software, e.g., in response a CT-FFR value satisfying or failing to satisfy a preset FFR threshold such as an FFR value indicative of severe stenosis.

The GUI 802 further includes a tool bar 822 with a plurality of software based tools, including a first tool 824₁, ..., and an Mth tool 824_{M} where M is a positive integer equal to or greater than one. Examples of software based tools include a measurement tool, an annotation tool, a statistics tool, a zoom tool, a pan tool, a rotate tool, a window/level tool, a screenshot tool, a rendering tool, etc. One or more of the software based tools can be used with one or more of the N viewports. In one instance, the measurement tool is utilized to acquire the diameter information shown in the annotations 806, 808, 818 and 820. Additionally, or alternatively, the annotation tool is utilized to add the annotations 806, 808, 818 and 820. In one instance, the tool bar 822 further includes tools for opening additional viewports, rearranging the viewport layout, changing a size of a viewport, etc.

As briefly described herein, the composite rendering can be combined with other information. FIGURE 9 graphically depicts an example of a single rendering that includes a composite rendering 902 combined with image data of a heart 904. The composite rendering 902, as described herein, includes a coronary tree segmentation 906, plaque segmentations 908, 910, 912, 914, 916 and 918, and an FFR value mapping (gray scale here) superimposed or overlaid over the coronary tree segmentation 906. In this example, the image data of the heart 904 includes the myocardium and/or one or more chambers of the heart, and the combined rendering provides the full anatomical landscape, etc.

FIGURE 10 graphically depicts another example of a single rendering that includes a composite rendering 1002 combined with image data of a heart 1004. The composite rendering 1002, as described herein, includes a coronary tree segmentation 1006, plaque segmentations 1008, 1010 and 1012, and an FFR value mapping (gray scale here) superimposed or overlaid over the coronary tree segmentation 1006, with a CT-FFR value 1014 displayed at a region of interest, using the approach described herein and/or other approach. In this example, the image data of the heart 904 includes the subtended volume of the heart that is fed by each vessel, and can be used to guide treatment strategy, etc.

FIGURE 11 illustrates a non-limiting example of a flow chart for generating a composite rendering that includes a coronary artery tree segmentation, plaque segmentations, and CT-FFR information, in accordance with an aspect herein. It is to be appreciated that the ordering of the acts in the method is not limiting. As such, other orderings are contemplated herein. In addition, one or more acts may be omitted, and/or one or more additional acts may be included.

At 1102, CT image data is obtained, as described herein and/or otherwise. For example, in one instance the imaging system 102 is employed to perform a CCTA and/or other imaging examination and reconstruct CT image data. In this instance, the CT image data is obtained from the imaging system 102 (e.g., from the reconstructor 136 and/or the memory 150) and/or the remote resource 146. In another instance, the CT image data is obtained from a different imaging system.

At 1104, a coronary artery tree is segmented from the CT image data, as described herein and/or otherwise. For example, this includes automated and/or semi-automated approaches to identify and/or extract coronary artery centerlines and/or lumen geometry based on voxel intensity, object shape, and/or other characteristics. At 1106, plaque is segmented from the CT image data and/or segmented coronary artery tree, as described herein and/or otherwise. This includes classifying plaque segmentations as calcified, non-calcified or mixed plaque.

At 1108, FFR information, such as FFR values, is determined for one or more vessels of the segmented coronary artery tree, as described herein and/or otherwise. Again, FFR values are values from 0.0 to 1.0, where a value 0.80 indicates functional significance, and a value of 0.70 indicates severe pathological blood pressure. Again, this may include employing a CFD module to compute a transstenotic pressure difference, subtracting the transstenotic pressure difference from the aortic pressure to estimate the distal coronary pressure, and computing an FFR based on these pressures.

At 1110, a composite rendering is generated based on the segmentation of the coronary tree, the segmentation of the plaque and the FFR information, as described herein and/or otherwise. For example, and as depicted in FIGURE 5, the composite rendering can include a rendering of an interior of the coronary tree with indicia corresponding to FFR value mapped along the interior of the vessels and plaque segmentations disposed over the interior of the vessels at locations where plaque resides within the vessels. In another example, as depicted in FIGURE 6, a cut mask can be employed at regions of plaque to visualize the inside of the vessels where the plaque segmentations are disposed at the plaque locations in the vessels.

At 1112, the composite rendering is displayed, as described herein and/or otherwise. For example, in one instance the composite rendering is displayed by itself. In another instance, the composite rendering is displayed within a viewport of graphical user interface, e.g., as depicted in FIGURE 8, with or without one or more over viewports displaying other information such as image slices from the image data, curved MPR, and/or other renderings. In another example, the composite rendering can be merged with at least a subset of other image data, as depicted in FIGURES 9 and 10. In any instance, the system can be configured such that hovering a pointer over a region of a vessel invokes display of the FFR value corresponding to the region.

FIGURE 12 illustrates another non-limiting example of a flow chart for generating a composite rendering that includes a coronary artery tree segmentation, plaque segmentations, and CT-FFR information, in accordance with an aspect herein. It is to be appreciated that the ordering of the acts in the method is not limiting. As such, other orderings are contemplated herein. In addition, one or more acts may be omitted, and/or one or more additional acts may be included.

At 1202, a coronary artery tree segmentation is obtained, as described herein and/or otherwise. At 1204, one or more plaque segmentations are obtained for the coronary tree segmentation, as described herein and/or otherwise. At 1206, FFR information is obtained for coronary tree segmentation, as described herein and/or otherwise. At 1208, a composite rendering is generated with the coronary tree segmentation, the one or more plaque segmentations, and the FFR information for the coronary tree segmentation, as described herein and/or otherwise.

The composite rendering can be displayed, as described herein and/or otherwise. For example, in one instance the composite rendering is displayed alone. In another instance, the composite rendering is displayed with other image data. In this instance, the composite rendering displayed can be displayed in its own viewport separate from one or more viewports displaying the other image data and/or combined in a viewport with some of the other image data. In any instance, the FFR value for a particular region of a vessel of the coronary tree can be determined by hovering a pointer over the region, which results in the corresponding FFR value.

The above can be implemented by way of computer readable instructions, encoded, or embedded on the computer readable storage medium, which, when executed by a computer processor, cause the processor to carry out the described acts or functions. Additionally, or alternatively, at least one of the computer readable instructions is carried out by a signal, carrier wave or other transitory medium, which is not computer readable storage medium.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include such additional elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

The various embodiments and/or components, for example, the modules, or components and controllers therein, also may be implemented as part of one or more computers or processors. The computer or processor may include a computing device, an input device, a display unit and an interface, for example, for accessing the Internet. The computer or processor may include a microprocessor. The microprocessor may be connected to a communication bus. The computer or processor may also include a memory. The memory may include Random Access Memory (RAM) and Read Only Memory (ROM). The computer or processor further may include a storage device, which may be a hard disk drive or a removable storage drive such as a floppy disk drive, optical disk drive, and the like. The storage device may also be other similar means for loading computer programs or other instructions into the computer or processor.

As used herein, the term "computer" or "module" may include any processor-based or microprocessor-based system including systems using microcontrollers, reduced instruction set computers (RISC), application specific integrated circuits (ASICs), logic circuits, and any other circuit or processor capable of executing the functions described herein. The above examples are exemplary only, and are thus not intended to limit in any way the definition and/or meaning of the term "computer". The computer or processor executes a set of instructions that are stored in one or more storage elements, in order to process input data. The storage elements may also store data or other information as desired or needed. The storage element may be in the form of an information source or a physical memory element within a processing machine.

The set of instructions may include various commands that instruct the computer or processor as a processing machine to perform specific operations such as the methods and processes of the various embodiments of the invention. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

As used herein, the terms "software" and "firmware" are interchangeable, and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the invention without departing from their scope. While the dimensions and types of materials described herein are intended to define the parameters of the various embodiments of the invention, the embodiments are by no means limiting and are exemplary embodiments. Many other embodiments will be apparent to those of skill in the art upon reviewing the above description.

This written description uses examples to disclose the various embodiments of the invention, including the best mode, and also to enable any person skilled in the art to practice the various embodiments of the invention, including making and using any devices or systems and performing any incorporated methods. The patentable scope of the various embodiments of the invention is defined by the claims, and may include other examples that occur to those skilled in the art. Such other examples are intended to be within the scope of the claims if the examples have structural elements that do not differ from the literal language of the claims, or if the examples include equivalent structural elements with insubstantial differences from the literal languages of the claims.

Embodiments of the present disclosure shown in the drawings and described above are example embodiments only and are not intended to limit the scope of the appended claims, including any equivalents as included within the scope of the claims. Various modifications are possible and will be readily apparent to the skilled person in the art. It is intended that any combination of non-mutually exclusive features described herein are within the scope of the present disclosure. That is, features of the described embodiments can be combined with any appropriate aspect described above and optional features of any one aspect can be combined with any other appropriate aspects. Similarly, features set forth in dependent claims can be combined with non-mutually exclusive features of other dependent claims, particularly where the dependent claims depend on the same independent claim. Single claim dependencies may have been used as practice in some jurisdictions that require them, but this should not be taken to mean that the features in the dependent claims are mutually exclusive.

## Claims

1. A computer-implemented method, comprising:
obtaining an image of one or more anatomical vessels of a subject (1202), wherein the image is a segmentation of the one or more anatomical vessels from image data acquired with a three-dimensional medical imaging modality;
obtaining one or more segmentations of plaque in the one or more anatomical vessels of the subject (1204);
obtaining Fractional Flow Reserve (FFR) information for the one or more anatomical vessels of the subject (1206);
generating a composite rendering that includes the image of one or more anatomical vessels, at least one of the one or more segmentations of plaque, and the FFR information (1208); and
displaying the composite rendering in a single viewport.

2. The computer-implemented method of claim 1, further comprising:
visually mapping the FFR information to the one or more anatomical vessels with graphical indicia.

3. The computer-implemented method of claim 2, further comprising:
visually displaying an FFR value at a region of interest of the one or more anatomical vessels.

4. The computer-implemented method of claim 1, wherein the composite rendering includes a rendering of an interior of the one or more anatomical vessels, and further comprising:
superimposing a plaque segmentation of the one or more segmentations of plaque on a location of the interior of a vessel of the one or more anatomical vessels where a corresponding plaque is within the vessel.

5. The computer-implemented method of claim 1, wherein the rendering includes a rendering of the one or more anatomical vessels, and further comprising:
displaying an interior of a region of the one or more anatomical vessels at a cut out in the outer surface at a location where a plaque is located; and
superimposing a plaque segmentation corresponding to the plaque over the interior of the region.

6. The computer-implemented method of claim 1, further comprising:
concurrently displaying other image data in one or more other viewports.

7. The computer-implemented method of claim 6, further comprising:
displaying at least a subset of the other image data merged with the FFR information in a viewport of the one or more viewports.

8. The computer-implemented method of claim 1, further comprising:
combining the composite rendering with other image data in the single viewport.

9. The computer-implemented method of claim 1, further comprising:
at least one of segmenting the one or more anatomical vessels from image data to generate the image, segmenting plaque in the one or more anatomical vessels to generate the one or more segmentations of plaque, and determining the FFR information for the one or more anatomical vessels from the image data.

10. The computer-implemented method of claim 1, further comprising:
acquiring the image data with the three-dimensional medical imaging modality.

11. A system (102), comprising:
a medical imaging modality (104) configured to acquire projection data and generate three-dimensional image data of an interior region of a subject;
a display device (144); and
an operator console (138) with a processor (148) configured to:
segment the three-dimensional image data to generate an image of one or more anatomical vessels of a subject;
segment the three-dimensional image data or the image to generate one or more segmentations of plaque in the one or more anatomical vessels;
determine FFR information for the one or more anatomical vessels from the three-dimensional image data;
generate a composite rendering that includes the image of one or more anatomical vessels, the one or more segmentations of plaque, and the FFR information; and
display the composite rendering in a single viewport visually presented on the display device.

12. The system of claim 11, wherein the processor is further configured to visually map the FFR information to the one or more anatomical vessels with graphical indicia.

13. The system of claim 12, wherein the processor is further configured to visually display an FFR value for a region of interest of the one or more anatomical vessels with graphical indicia.

14. The system of claim 12, wherein the processor is further configured to display an interior of a region of the one or more anatomical vessels where a plaque is located and superimpose a plaque segmentation corresponding to the plaque over the interior of the region.

15. A computer readable medium (150) encoded with computer executable instructions, which, when executed by a processor, causes the processor to:
acquire projection data and generate three-dimensional image data of an interior region of a subject with a medical imaging modality;
segment an image of one or more anatomical vessels of the subject from the three-dimensional image data;
segment one or more segmentations of plaque in the one or more anatomical vessels from the three-dimensional image data or the image;
determine FFR information for the one or more anatomical vessels with the three-dimensional image data;
generate a composite rendering that includes the image of one or more anatomical vessels, the one or more segmentations of plaque, and the FFR information; and
display the composite rendering in a single viewport visually presented on a display device.
